(19) Europäisches Patentamt
European Patent Office
Office européen des brevets

(11) **EP 4 136 979 A1**

(12) **EUROPEAN PATENT APPLICATION**
published in accordance with Art. 153(4) EPC

(43) Date of publication:
**22.02.2023 Bulletin 2023/08**

(21) Application number: **21787957.6**

(22) Date of filing: **12.04.2021**

(51) International Patent Classification (IPC):
*A23C 9/127* (2006.01)    *A23C 9/13* (2006.01)
*C12N 15/55* (2006.01)    *C12N 9/16* (2006.01)

(52) Cooperative Patent Classification (CPC):
**A23C 9/127; A23C 9/13; C12N 9/14; C12N 9/16**

(86) International application number:
**PCT/JP2021/015188**

(87) International publication number:
**WO 2021/210539 (21.10.2021 Gazette 2021/42)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **13.04.2020 JP 2020071708**
                **13.04.2020 JP 2020071709**

(71) Applicant: **Godo Shusei Co., Ltd.
Matsudo-shi, Chiba 271-0064 (JP)**

(72) Inventors:
• **OGASAWARA, Junki
  Matsudo-shi, Chiba 271-0064 (JP)**
• **HAMAGUCHI, Kazuhiro
  Matsudo-shi, Chiba 271-0064 (JP)**

(74) Representative: **Hartz, Nikolai
  Wächtershäuser & Hartz
  Patentanwaltspartnerschaft mbB
  Weinstrasse 8
  80333 München (DE)**

Remarks:
The complete document including Reference
Table(s) and the Sequence Listing(s) can be
downloaded from the EPO website

(54) **FERMENTED MILK, MANUFACTURING METHOD THEREFOR, AND DEPHOSPHORYLATED MILK**

(57)    Provided is fermented milk having stable quality and a method for the manufacture thereof, and a dephosphorylated milk.
   The present invention relates to fermented milk containing 0.8 g / 100 g or more of dephosphorylated casein.

EP 4 136 979 A1

**Description**

Technical Field

The present invention relates to fermented milk, a method for manufacturing the fermented milk, and dephosphorylated milk.

Background Art

Fermented milk is manufactured by fermenting raw material milk with microorganisms such as lactic acid bacteria. In recent years, physiological functions of extracellular polysaccharides (EPS) extracellularly produced by lactic acid bacteria during fermentation have attracted attention.

A main protein of milk is casein. When fermentation decreases pH, casein micelles aggregate to one another to form a gel. Casein is mainly classified into four types, namely, $\alpha_{s1}$, $\alpha_{s2}$, $\beta$, and $\kappa$, and these types contain phosphoric acids bound to 8 serine residues, 11 serine residues, 5 serine residues, and one serine residue, respectively. It is known that the phosphoric acid modification in casein affects the hydrophobic level of a casein micelle surface or a hydrophobic core.

The organization (physical properties) of fermented milk changes over time. In particular, water weeping (isolation of whey) under storage is one of factors that reduce the commercial values of the fermented milk. Therefore, in order to prevent water weeping, a stabilizer such as pectin and a functional milk raw material, such as a milk protein concentrate have been made use of. Furthermore, usage of an enzyme has been studied. For example, a method of using, for example, an enzyme having a milk-coagulating activity such as transglutaminase or glucose oxidase has been reported (for example, see Patent Literature 1).

However, a method for manufacturing milk and fermented milk by using an enzyme that acts on phospho-serine of casein has not been known.

Citation List

Patent Literature

Patent Literature 1: WO 2015/041194

Summary of the Invention

Technical Problem

An object of the present invention is to provide fermented milk having stable quality, a method for manufacturing the fermented milk, and dephosphorylated milk.

Solution to Problem

The inventors focused on and earnestly studied about phospho-serine of casein, and found that dephosphorylated casein resulting from elimination of a phosphoric acid from a serine residue of casein affected physical properties of fermented milk; water weeping was substantially prevented in the fermented milk containing a predetermined amount of the dephosphorylated casein; viscosity and breaking stress increased; and the production amount of extracellular polysaccharides (EPS) increased. Furthermore, the inventors found that, by fermenting raw material milk in the presence of protein phosphatase or fermenting raw material milk enzyme-treated with protein phosphatase, water weeping was substantially prevented in the fermented milk; viscosity and breaking stress increased; and the production of extracellular polysaccharides (EPS) was enhanced, and thus the inventors accomplished the present invention.

Specifically, the present invention provides the following [1] to [6].

[1] Fermented milk, containing 0.8 g / 100 g or more of dephosphorylated casein.
[2] The fermented milk according to [1], wherein a dephosphorylated casein content is 0.8 g / 100 g to 1.5 g / 100 g.
[3] The fermented milk according to [1] or [2], wherein a free phosphoric acid content is 15 mM or more.
[4] The fermented milk according to any one of [1] to [3], the fermented milk being yogurt.
[5] Dephosphorylated milk, containing dephosphorylated casein.
[6] The dephosphorylated milk according to [5], a dephosphorylated casein content is 0.8 g / 100 g or more.
Furthermore, the present invention provides the following [7] to [13].

[7] A method for manufacturing fermented milk, the method including any one of the following steps:

(a) fermenting raw material milk in the presence of protein phosphatase; and
(b) fermenting raw material milk enzyme-treated with protein phosphatase.

[8] The method for manufacturing the fermented milk according to [7], wherein the protein phosphatase is protein phosphatase derived from a microorganism belonging to the genus Trichoderma.
[9] The method for manufacturing the fermented milk according to [8], wherein the microorganism belonging to the genus Trichoderma is Trichoderma virens.
[10] The method for manufacturing the fermented milk according to any one of [7] to [9], wherein the protein phosphatase is any one of the following (i) to (iii):

(i) a protein comprising an amino acid sequence of SEQ ID NO: 2;
(ii) a protein comprising an amino acid sequence of SEQ ID NO: 2 in which one or several amino acids are deleted, substituted, or added, the protein having protein phosphatase activity; and
(iii) a protein comprising an amino acid sequence having at least 80% sequence identity with an amino acid sequence of SEQ ID NO: 2, the protein having protein phosphatase activity.

[11] The method for manufacturing the fermented milk according to any one of [7] to [10], wherein the fermented milk is yogurt.
[12] An enzyme agent for fermented milk, the enzyme agent containing protein phosphatase.
[13] A protein having protein phosphatase activity, the protein being any one of the following (i) to (iii):

(i) a protein comprising an amino acid sequence of SEQ ID NO: 2;
(ii) a protein comprising an amino acid sequence of SEQ ID NO: 2 in which one or several amino acids are deleted, substituted, or added, the protein having protein phosphatase activity; and
(iii) a protein comprising an amino acid sequence having at least 80% sequence identity with an amino acid sequence of SEQ ID NO: 2, the protein having protein phosphatase activity.

Advantageous Effects of Invention

The fermented milk according to the present invention exhibits less water weeping and has a highly-viscous and firm organization. Furthermore, the fermented milk contains many extracellular polysaccharides (EPS) and has an excellent physiology function.
The method for manufacturing fermented milk according to the present invention facilitates to provide fermented milk that exhibits less water weeping, has a highly-viscous and firm organization, and contains many extracellular polysaccharides (EPS).
Dephosphorylated milk of the present invention changes in mouthfeel owing to an increase in the hydrophobic level of a casein molecule. The above-mentioned effect can be achieved also in a dairy product using the dephosphorylated milk.

Brief Description of Drawings

FIG. 1 is a schematic diagram illustrating the electrophoresis of cow's milk.
FIG. 2 is a diagram illustrating a method for measuring phosphorylated casein and dephosphorylated casein.
FIG. 3 is a diagram illustrating a. temperature dependency, b. temperature stability, c. pH dependency, and d. pH stability of protein phosphatase derived from Trichoderma virens Gv29-8.
FIG. 4 is a diagram illustrating the metal ion requirement of protein phosphatase derived from Trichoderma virens Gv29-8.
FIG. 5 is a diagram illustrating the concentration of free phosphoric acid in fermented milk of Example 1.
FIG. 6 is a diagram illustrating the concentration of free phosphoric acid in dephosphorylated milk of Example 2.
FIG. 7 is a diagram illustrating the water weeping percentage (%), the free phosphoric acid concentration (mM), and the pH of fermented milk to which protein phosphatase derived from Trichoderma virens Gv29-8 is added.
FIG. 8 is a diagram illustrating the water weeping percentage (%), the free phosphoric acid concentration (mM), and the pH of fermented milk to which protein phosphatase derived from Trichoderma virens Gv29-8 is added.
FIG. 9 is a diagram illustrating the amount ($\mu$g/g-fermented milk) of extracellular polysaccharides (EPS) in fermented milk to which protein phosphatase derived from Trichoderma virens Gv29-8 is added.

FIG. 10 is a diagram illustrating the viscosity (cP) of fermented milk to which protein phosphatase derived from Trichoderma virens Gv29-8 is added.

FIG. 11 is a diagram illustrating analysis results on the breaking strength of fermented milk to which protein phosphatase derived from Trichoderma virens Gv29-8 is added.

FIG. 12 is a diagram illustrating the water weeping percentage (%), the free phosphoric acid concentration (mM), and the pH of fermented milk to which protein phosphatase derived from Trichoderma virens Gv29-8 is added.

FIG. 13 is a diagram illustrating the amount (μg/g-fermented milk) of extracellular polysaccharides (EPS) in fermented milk to which protein phosphatase derived from Trichoderma virens Gv29-8 is added.

FIG. 14 is a diagram illustrating the viscosity (cP) of fermented milk to which protein phosphatase derived from Trichoderma virens Gv29-8 is added.

FIG. 15 is a diagram illustrating analysis results on the breaking strength of fermented milk to which protein phosphatase derived from Trichoderma virens Gv29-8 is added.

FIG. 16 is a diagram illustrating the water weeping percentage (%), the free phosphoric acid concentration (mM), and the pH of fermented milk to which protein phosphatase derived from Trichoderma virens Gv29-8 is added.

FIG. 17 is a diagram illustrating the amount (μg/g-fermented milk) of extracellular polysaccharides (EPS) in fermented milk to which protein phosphatase derived from Trichoderma virens Gv29-8 is added.

FIG. 18 is a diagram illustrating the viscosity (cP) of fermented milk to which protein phosphatase derived from Trichoderma virens Gv29-8 is added.

FIG. 19 is a diagram illustrating analysis results on the breaking strength of fermented milk to which protein phosphatase derived from Trichoderma virens Gv29-8 is added.

FIG. 20 is a diagram illustrating the water weeping percentage (%), the free phosphoric acid concentration (mM), and the pH of fermented milk obtained by fermenting raw material milk treated with protein phosphatase derived from Trichoderma virens Gv29-8.

FIG. 21 is a diagram illustrating changes in the pH of fermented milk to which protein phosphatase derived from Trichoderma virens Gv29-8 is added.

FIG. 22 is a diagram illustrating the water weeping percentage (%), the free phosphoric acid concentration (mM), and the pH of fermented milk to which protein phosphatase derived from Trichoderma virens Gv29-8 is added.

FIG. 23 is a diagram illustrating changes in the concentration of free phosphoric acid and the concentration of calcium ions in raw material milk treated with protein phosphatase derived from Trichoderma virens Gv29-8.

Detailed Description of the Invention

In the present specification, fermented milk encompasses fermented milk and lactic acid bacteria beverages defined in the ministerial ordinance ("Ministerial Ordinance on Milk and Milk products Concerning Compositional Standards, etc.", Ministry of Health and Welfare Ordinance No. 52, 1951). Here, fermented milk is defined as "the products which are obtained by fermenting milk, or milk or the like containing an equal or greater amount of non-fat-milk solids with lactic acid bacteria or yeast and then forming a paste or liquid, or the frozen product". Lactic acid bacteria beverages are defined as "the drinks which are obtained by fermenting milk or the like with lactic acid bacteria or yeast and then processing the resultant or using the resultant as a principal ingredient (excluding fermented milk) ".

Examples of the fermented milk classified as the "paste product" include hard yogurt and soft yogurt. Examples of the fermented milk classified as the "liquid product" include drinkable yogurt. Examples of the fermented milk classified as the "frozen product" include frozen yogurt. From the viewpoint of ease of achieving the effect of the present invention, the fermented milk according to the present invention is preferably hard yogurt or soft yogurt.

The fermented milk according to the present invention contains 0.8 g / 100 g or more of dephosphorylated casein. The dephosphorylated casein is produced by elimination of serine phosphate of casein in milk.

From the viewpoints of the effect of substantially preventing water-weeping in the fermented milk, the effect of increasing viscosity and breaking stress, and the effect of increasing EPS production volume, the amount of the dephosphorylated casein contained in the fermented milk is preferably 0.9 g / 100 g or more and more preferably 1.0 g / 100 g or more, and preferably 1.5 g / 100 g or less, more preferably 1.4 g / 100 g or less, and still more preferably 1.3 g / 100 g or less.

In the fermented milk according to the present invention, the ratio of the dephosphorylated casein in casein is preferably 25% to 45%, and more preferably 26% to 43%.

In the present specification, an analysis of casein can be performed in such a manner that each type of casein (α casein, β casein, and κ casein) having a known concentration and a sample are subjected to polyacrylamide gel electrophoresis (SDS-PAGE) and Coomassie Brilliant Blue staining (CBB staining), and then the strength of a band is converted into numbers by using an image analysis software to produce a calibration curve, whereby the amount of the casein in the sample can be calculated.

In the present specification, an analysis of the dephosphorylated casein shall follow the method described below.

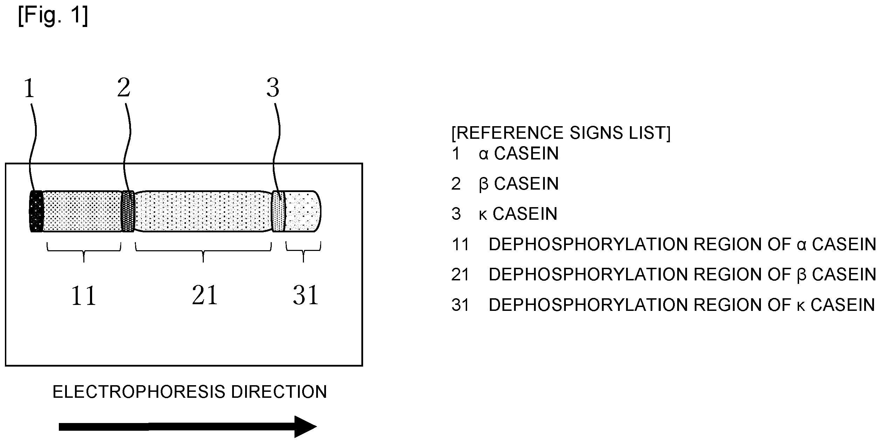

The dephosphorylated casein can be quantified by an electrophoresis method. According to the electrophoresis method, the amount of phosphorylated casein and the amount of dephosphorylated casein are calculated from electrophoresis results. The method will be described using FIG. 1. Note that the electrophoresis method is as described in Examples below.

FIG. 1 schematically illustrates the electrophoresis of cow's milk. Phosphorylated casein includes three regions, namely, α casein 1, β casein 2, and κ casein 3, and the amount of the phosphorylated casein can be calculated by integrating these three. Other types of phosphorylated casein are also present, but in small amounts, and therefore can be ignored. Note that the total of αs-1 casein, αs-2 casein, β casein, and κ casein accounts for approximately 97% of all types of casein in the cow's milk.

Dephosphorylated casein is adjacent to a band of each casein. Since there are various levels of dephosphorylation in the dephosphorylated casein, the dephosphorylated casein becomes smeared after electrophoresis. The band of the phosphorylated casein and the region of the dephosphorylated casein have different concentrations to each other, and therefore these can be visually identified. By integrating dephosphorylated casein regions 11, 21, and 31, the amount of the dephosphorylated casein can be calculated. An end of the dephosphorylated casein region 31 is set by visually identifying the boundary between a stained region and a non-stained region.

When cow-derived milk is used, the amount of phosphorylated casein is found to be smaller in the order of α casein, β casein, and κ casein. The amount of dephosphorylated casein in milk having different origins can be also measured using the same method as the above-described method. At least one specific casein is beneficially selected and measured so that the specific casein accounts for not less than 90% of the amount of all types of casein.

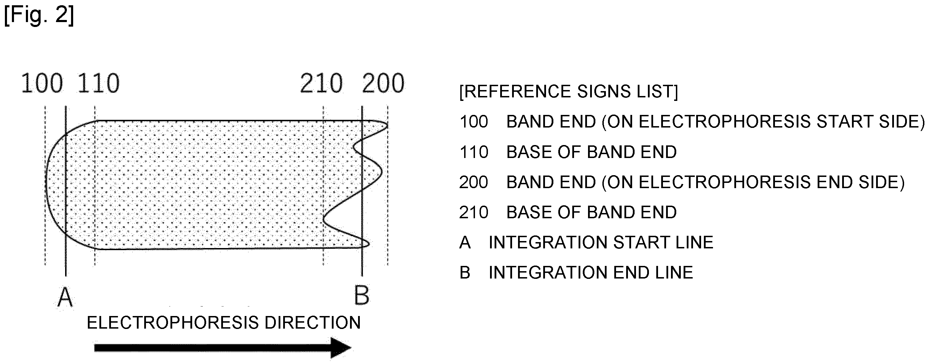

A band illustrated in Fig. 1 has a good shape, but, actually a band often has a distorted shape even under calibration of various conditions. A method for measuring the phosphorylated casein and the dephosphorylated casein in this case will be described using FIG. 2.

When a non-band region is present between a band end 100 (on an electrophoresis start side) and a base 110 of the band end in FIG. 2, an integration start line A is set at a point where the area of the non-band region visually accounts for 50%, and the area of a band region extending from the integration start line A is calculated. For the setting of the integration start line A, a graphically expressed electrophoresis pattern may be referred.

When a non-band region is present between a band end 200 (on an electrophoresis end side) and a base 210 of the band end in FIG. 2, an integration end line B is set at a point where the area of the non-band region visually accounts for 50%, and the area of a band region extending to the integration end line B is calculated. For the setting of the integration end line B, a graphically expressed electrophoresis pattern may be referred to.

It is beneficial that the integration start line and the integration end line be set using the above-described method, and a concentration in the band region present between the lines is quantitatively determined.

A concentration in a dephosphorylation region can be quantitatively determined in the same manner as the method described above.

The fermented milk according to the present invention preferably further contains free phosphoric acid.

From the viewpoints of the effect of substantially preventing water-weeping in the fermented milk, the effect of increasing viscosity and breaking stress, and the effect of increasing EPS production volume, the amount of free phosphoric acid contained in the fermented milk is preferably 15 mM or more, and more preferably in a range of 15 mM to 40 mM.

In order to make the fermented milk contain the dephosphorylated casein, for example, raw material milk as a raw material of the fermented milk may be beneficially fermented in the presence of protein phosphatase. Alternatively, prior to the fermentation of raw material milk, the raw material milk may be treated with protein phosphatase, and after the treatment, the resultant dephosphorylated milk may be beneficially fermented.

Namely, the method for manufacturing the fermented milk according to the present invention includes any one of the steps of:

(a) fermenting raw material milk in the presence of protein phosphatase; and
(b) fermenting raw material milk enzyme-treated with protein phosphatase.

The raw material milk beneficially contains casein, and a common milk-derived raw material may be used as the raw material milk. Examples of the raw material milk include raw milk, cow's milk, special milk, raw goat's milk, pasteurized goat's milk, raw sheep's milk, composition modified milk, low fat milk, skimmed milk, processed milk, cream, butter, butter oil, cheese, concentrated whey, ice creams, concentrated milk, concentrated skimmed milk, evaporated milk, evaporated skimmed milk, sweetened condensed milk, sweetened condensed skimmed milk, whole milk powder, skimmed milk powder, cream powder, whey powder, protein concentrated whey powder, and buttermilk powder.

The amount of casein contained in the raw material milk is not particularly limited, but is preferably 0.01g to 10g/100g, more preferably 0.1 g to 8 g / 100 g, and still more preferably 1 g to 5 g / 100 g.

The raw material milk has preferably a pH of 4.0 to 8.0, more preferably a pH of 4.7 to 7.5, and still more preferably a pH of 5.3 to 6.8. The same goes to the pH of raw material milk to which the following components other than the raw material milk are added.

The raw material milk may contain other components that can be blended before or after fermentation, as needed, within a range of not impairing advantageous effects of the present invention. Examples of the other components include sweeteners (such as monosaccharide, oligosaccharide, sugar alcohols, and artificial sweeteners), stabilizers (such as gelatin, pectin, carrageenan, and xanthan gum), fruit juices, fruit fleshes, and flavors.

Protein phosphatase is an enzyme capable of dephosphorylating phosphorylated proteins. In the present specification, protein phosphatase may beneficially be capable of eliminating serine phosphate of casein in raw material milk. The protein phosphatase preferably eliminates the serine phosphate of the casein in the raw material milk during fermentation.

The best (optimum) pH of the protein phosphatase is preferably in a range of from 4.0 to 7.5, more preferably in a range of from 4.5 to 7.0, and still more preferably in a range of from 5.0 to 6.0.

The protein phosphatase is preferably gradually deactivated during the manufacture of the fermented milk and is in a deactivated state in the fermented milk. Specifically, the protein phosphatase preferably acts even under the condition of the weak acidity of pH 5.0 to 6.0 and is deactivated under the condition of less than pH 4.5. During the fermentation of the raw material milk, the phosphoric acid is gradually eliminated from the casein. It can be considered that, through the process in which free phosphoric acid in milk increases in amounts and lactic acid bacteria metabolize the free phosphoric acid, EPS is more easily produced. The protein phosphatase is preferably deactivated after the completion of fermentation because the quality of the fermented milk is stably maintained.

The optimum temperature of the protein phosphatase is preferably in a range of 1°C to 60°C, and more preferably in a range of 10°C to 55°C.

The protein phosphatase used in the present invention preferably has the above-mentioned optimum pH and the above-mentioned optimum temperature. The type and the origin of the protein phosphatase are not particularly limited, but are preferably protein phosphatase derived from a microorganism belonging to the genus Trichoderma, Aspergillus, Sccharomyces, Bacillus, or Streptomyces, more preferably protein phosphatase derived from Trichoderma virens, and still more preferably a virtual protein derived from Trichoderma virens Gv29-8 (XP_013951069.1 hypothetical protein TRIVIDRAFT_87714). The virtual protein (XP_013951069.1 hypothetical protein TRIVIDRAFT_87714) has protein phosphatase activity having been made clear by the inventors, and has the following properties.

(a) Optimum temperature: 50°C
(b) Temperature stability: 80% or higher of remaining activity at 43°C for 2 hours.
(c) Optimum pH: pH 5.42
(d) pH stability: 80% or higher of remaining activity at pH 4.7 to 6.8 for 6 hours.
(e) Metal Ion Requirement: activated with 2.5 to 5.0 mM of bivalent metal cations, such as $Ca^{2+}$, $Mg^{2+}$, $Mn^{2+}$, or $Co^{2+}$

The base sequence of DNA encoding protein phosphatase derived from Trichoderma virens Gv29-8 (XP_013951069.1 hypothetical protein TRIVIDRAFT_87714) is represented by SEQ ID NO: 1 in the sequence listing, meanwhile the amino acid sequence is represented by SEQ ID NO: 2 in the sequence listing.

The protein phosphatase may be wild-type protein phosphatase or may be protein phosphatase in which a gene to encode the protein phosphatase is expressed in a host such as Escherichia coli or the above-mentioned gene is modified or expressed by various types of gene manipulation, the protein phosphatase having protein phosphatase activity.

The protein phosphatase is preferably any one of the following proteins (i) to (iii):

(i) a protein comprising an amino acid sequence of SEQ ID NO: 2;
(ii) a protein comprising an amino acid sequence of SEQ ID NO: 2 in which one or several amino acids are deleted, substituted, or added, the protein having protein phosphatase activity; and
(iii) a protein comprising an amino acid sequence having at least 80% sequence identity with an amino acid sequence of SEQ ID NO: 2, the protein having protein phosphatase activity.

The number of amino acids deleted, substituted, or added in the amino acid sequence of SEQ ID NO: 2 in which one or several amino acids are deleted, substituted, or added is not limited as long as enzyme activity equivalent to that of the protein phosphatase comprising the amino acid sequence of SEQ ID NO: 2 is exhibited, but the number of amino acids deleted, substituted, or added is preferably 1 to 20, more preferably 1 to 10, and still more preferably 1 to 8.

The sequence identity with the amino acid sequence of SEQ ID NO: 2 is 80% or higher, preferably 85% or higher, more preferably 90% or higher, still more preferably 95% or higher, and still more preferably 99% or higher. The percentage of sequence identity can be calculated using open or commercially available software having an algorithm for making comparison using a standard sequence as a reference sequence. Examples of the software that can be used include BLAST, FASTA, and GENETYX (manufactured by GENETYX CORPORATION).

The protein phosphatase used in the present invention is capable of stabilizing the quality of fermented milk and enhancing the physiology function of the fermented milk, and is therefore useful as an enzyme agent for fermented milk.

At the step (a), the protein phosphatase is beneficially present during the fermentation of the raw material milk. The timing for adding the protein phosphatase is not particularly limited.

The amount of the protein phosphatase used at the step (a) beneficially corresponds to a concentration that makes it possible to eliminate serine phosphate of casein in the raw material milk. For example, the amount of the protein phosphatase used is preferably from 0.1 to 25 U, more preferably from 0.5 to 15 U, and still more preferably from 1 to 10 U with respect to 1 mL of the raw material milk. Here, in the present specification, 1 U means the amount of an enzyme that liberates 1 μmol of phosphoric acid per 1 minute under the condition that, to a liquid substrate in which 20 mg of cow's milk casein is dissolved per 1 mL of a 20 mM MES-NaOH buffer solution having a pH of 6.0 and containing 10 mM Tris-HCl, an enzyme solution being 1/10 as much as the liquid substrate is added, and the resultant mixture is allowed to react at 37°C, and a reaction stop solution in an equivalent amount to the mixture is added thereto.

As a method for the fermentation at the step (a), a common method is applied. For example, a fermentation mixture prepared by mixing and dissolving the raw material milk and other raw materials is subjected to homogenization, heat sterilization, and cooling, and then, a microorganism (starter) and the protein phosphatase are added thereto, followed by fermentation. After the cooling, the resultant mixture may be crushed and homogenized as needed.

The fermentation temperature is beneficially a temperature at which the microorganism (starter) grows and the enzyme is not deactivated. The fermentation temperature can be suitably set in accordance with the kinds thereof and is suitably in a range of from 20°C to 45°C, and more preferably from 30°C to 37°C.

The fermentation time is, for example, from 1 to 48 hours, preferably from 2 to 24 hours, more preferably from 3 to 10 hours, still more preferably from 3 to 6 hours, and particularly preferably from 3 to 5 hours.

The fermented milk preferably has a pH of from 4.0 to 5.0.

The raw material milk (dephosphorylated milk) enzyme-treated with the protein phosphatase that is used for the fermentation at the step (b) is beneficially such that casein in the raw material milk is dephosphorylated by the protein phosphatase. When the raw material milk is enzyme-treated with the protein phosphatase in advance, a larger amount of casein can be dephosphorylated and furthermore the degree of dephosphorylation of the casein can be easily controlled.

The amount of the protein phosphatase used at the step (b) beneficially corresponds to a concentration that makes it possible to eliminate serine phosphate of casein in the raw material milk. For example, the amount of the protein phosphatase used is preferably from 0.1 to 25 U, more preferably from 0.5 to 15 U, and still more preferably from 1 to 10 U with respect to 1 mL of the raw material milk.

The enzyme treatment of the raw material milk is preferably performed at a reaction temperature of 1°C to 60°C for a reaction time of from 0.1 to 24 hours. The reaction temperature is preferably from 10°C to 60°C, more preferably from 30°C to 55°C, and still more preferably from 40°C to 50°C. The reaction time is preferably from 0.3 to 12 hours, more preferably from 0.5 to 5 hours, and still more preferably from 1 to 4 hours.

Prior to the enzyme treatment of the raw material milk with the protein phosphatase, the pH of the raw material milk may be adjusted.

After the enzyme treatment, the raw material milk may be fermented without deactivating the enzyme, or the raw material milk may be fermented after treatment for deactivating the enzyme by heating or the like. It is preferable to ferment the raw material milk without deactivating the enzyme. Even during the fermentation, the dephosphorylation of casein gradually progresses.

The heating in the treatment for deactivating the enzyme is preferably performed at a high temperature in a short time, for example, preferably at from 60°C to 100°C for from 1 minute to 30 minutes. After the raw material milk is enzyme-treated before sterilization of the raw material milk, the enzyme may be deactivated during the sterilization of the raw material milk.

As a method for the fermentation at the step (b), a common method is applied as with the step (a). For example, a fermentation mixture including the raw material milk enzyme-treated with the protein phosphatase and other raw materials is subjected to homogenization, heat sterilization, and cooling, and then, a microorganism (starter) is added thereto and the resultant mixture is fermented. After the cooling, the resultant mixture may be crushed and homogenized as needed.

The fermentation temperature may be suitably set in the same way as at the step (a), and is preferably from 20°C to 45°C, and more preferably from 30°C to 37°C.

The fermentation time is, for example, from 1 to 48 hours, preferably from 2 to 24 hours, more preferably from 3 to 10 hours, still more preferably from 3 to 6 hours, and particularly preferably from 3 to 5 hours.

The fermented milk preferably has a pH of from 4.0 to 5.0.

In the present invention, the fermented milk is preferably manufactured by the step (a) from the viewpoints of easily forming a uniform curd and achieving excellence in handling properties and costs.

By treating the raw material milk with the protein phosphatase, casein in the raw material milk is dephosphorylated to obtain dephosphorylated milk containing dephosphorylated casein. When the hydrophobic level of a casein molecule is increased by the dephosphorylation of the casein, the mouthfeel of the milk changes. By making the most use of such change in the mouthfeel, the dephosphorylated milk of the present invention can be applied to various types of dairy products besides the fermented milk, as with the raw material milk.

The amount of the dephosphorylated casein contained in the dephosphorylated milk of the present invention is preferably 0.8 g / 100 g or more, more preferably 0.9 g / 100 g or more, and still more preferably 1.0 g / 100 g or more, and preferably 1.5 g / 100 g or less, and more preferably 1.4 g / 100 g or less from the viewpoints of making mouthfeel better and achieving fermented milk with stable quality.

In the dephosphorylated milk of the present invention, the ratio of the dephosphorylated casein in the casein is preferably from 45% to 65%, and more preferably from 48% to 63%.

The dephosphorylated milk preferably further contains free phosphoric acid, and the amount of the free phosphoric acid contained in the dephosphorylated milk is preferably in a range of from 1 mM to 100 mM, more preferably in a range of from 5 mM to 50 mM, still more preferably in a range of from 10 mM to 40 mM, and still more preferably in a range of from 12 mM to 30 mM from the viewpoints of making mouthfeel better and achieving fermented milk with stable quality.

For the fermentation of the raw material milk and the fermentation of the raw material milk (dephosphorylated milk) enzyme-treated with the protein phosphatase, a starter, such as common lactic acid bacteria or yeast, is used.

Examples of the lactic acid bacteria include Lactobacillus bacteria (such as Lactobacillus casei and Lactobacillus acidophilus), Lactococcus bacteria (such as Lactococcus lactis), Leuconostoc bacteria (such as Leuconostoc mesenteroides), Enterococcus bacteria (such as Enterococcus faecalis), and Bifidobacterium bacteria (such as Bifidobacterium bifidum and Bifidobacterium breve).

Examples of the yeast include Saccharomyces yeasts (such as Saccharomyces cerevisiae).

The number of the lactic acid bacteria or the yeast (the live bacteria) contained in the starter is, for example, from $10^5$ to $10^{13}$ cfu/mL, preferably from $10^6$ to $10^{12}$ cfu/mL, more preferably from $10^7$ to $10^{11}$ cfu/mL, and still more preferably from $10^8$ to $10^{10}$ cfu/mL.

The amount of the starter used is not particularly limited, and can be suitably set in accordance with the type of the starter. The amount of the starter used is from 0.01% to 10% by mass, preferably from 0.1% to 10% by mass, and more preferably from 0.5% to 10% by mass with respect to the raw material milk and/or the dephosphorylated milk.

The fermented milk of the present invention may contain other components that can be blended before or after fermentation, as needed, within a range of not impairing advantageous effects of the present invention. Examples of the other components include sweeteners (such as monosaccharide, oligosaccharide, sugar alcohols, and artificial sweeteners), stabilizers (such as gelatin, pectin, carrageenan, and xanthan gum), fruit juices, fruit fleshes, and flavors.

As described in Examples later, the fermented milk of the present invention exhibits less water weeping, and is highly viscous and has sufficient solidity.

The viscosity of the fermented milk is preferably such that, at a point in time when the fermented milk is treated for 30 seconds by a measurement method described later, the viscosity is preferably 3,000 Pa·s or higher. The upper limit of the viscosity is not particularly limited, but is 10000 Pa·s, for example.

The solidity of the fermented milk is such that, as a value obtained by a breaking strength analysis described in Examples later, a breaking load is preferably 0.41 N or more, more preferably 0.45 N or more, and still more preferably 0.5 N or more. The upper limit of the breaking load is not particularly limited, but is 1.0 N, for example.

The fermented milk of the present invention contains a large amount of extracellular polysaccharides (EPS). The amount of EPS in the fermented milk may be 40 μg or more, 60 μg or more, 80 μg or more, 110 μg or more, and 180 μg or more per 1 g of the fermented milk. The upper limit of the amount of EPS is not particularly limited, but is 300 μg, for example.

Examples

Next, the present invention will be described in more detail with Examples, but the present invention is not limited by Examples.

Manufacture Example 1 Purification of Protein Phosphatase derived from Trichoderma virens Gv29-8 (hereinafter, referred to as "PPase")

Trichoderma virens NBRC 6355 strain was inoculated into a potato dextrose agar medium (manufactured by EIKEN CHEMICAL CO., LTD.), and aerobically cultured at 25°C for 3 days. A colony of production bacteria grown on the potato dextrose agar medium was cut out together with the agar medium approximately 5 mm square, and inoculated into a production medium (pH 4.0) including 5.0% sucrose, 2.0% TUBERMINE FV (manufactured by Roquette Japan K.K.), 0.3% calcium chloride, 0.1% magnesium sulfate, and 0.001% dipotassium hydrogen phosphate, and cultured with rotation and shaking for 4 days on the condition of 27°C and 220 r/min.

Approximately 700 mL of the resultant culture medium was subjected to solid-liquid separation through suction filtration using ADVANTEC No.2 filter paper (manufactured by ADVANTEC CO.,LTD.) and 3.0% KC-FLOCK (manufactured by Nippon Paper Industries Co., Ltd.), and the obtained filtrate was concentrated to approximately 1/10 of original volume by UF (AHP, manufactured by Asahi Kasei Corporation). Subsequently, PEG4000 (manufactured by NACALAI TESQUE, INC.) was added to the concentrate so as to achieve a final concentration of 15%, and dissolved, and then left to stand at 4°C overnight. Subsequently, centrifugation (using HIMAC CENTRIFUGE CR20B2, manufactured by Hitachi, Ltd.) was conducted for 20 minutes on the condition of 8000 r/min and 4°C, and the resultant supernatant was discarded. The obtained precipitate was dissolved in approximately 10 mL of a 20 mM acetic acid-potassium acetate buffer solution (pH 4.8), and the resultant solution was kept warm at 50°C for 1 hour, and then centrifuged (using LEGEND MICRO 21R, manufactured by Thermo Fisher Scientific K.K.) for 10 minutes on the condition of 14800 r/min and 4°C. The supernatant was collected as a purified protein.

Based on amino acid sequence information, the purified protein was identified as a virtual protein derived from Trichoderma virens Gv29-8 (XP_013951069.1 hypothetical protein TRIVIDRAFT_87714). This protein was functionally unknown.

It was confirmed that the purified protein had protein phosphatase (PPase) activity.

1) Measurement of PPase Activity

0.2 g of cow's milk casein (manufactured by CALBIOCHEM, Casein, Bovine Milk, Carbohydrate and Fatty Acid Free) was weighed out in a 20-mL beaker, and 2.0 mL of a 0.1 M Tris-HCl buffer solution (pH 9.0) and a small amount of distilled water were added thereto, and dissolved using a magnetic stirrer. Subsequently, 2.0 mL of a 0.2 M MES-NaOH buffer solution (pH 6.0) and approximately 10 mL of distilled water were added, and, using 1.0 N hydrochloric acid, the resultant solution was adjusted to have a pH of 6.0, and then the volume was fixed at 20 mL. The resultant was regarded as a liquid substrate. 450 μL of the liquid substrate was dispensed into a 1.5-mL Eppendorf tube, and 50 μL of an enzyme sample was added thereto. The resultant mixture was allowed to react exactly for 20 minutes. After the reaction, 500 μL of a reaction stop solution including 18.0 g of trichloroacetic acid, 18.0 g of acetic anhydride sodium, and 19.8 g of acetic acid per 1 L of the solution was added, and stirred violently, and centrifuged for 5 minutes on the condition of 15000 r/min and 4°C. 200 μL of a suitably diluted solution of the resultant supernatant was added to 800 μL of distilled water, and subsequently 2 M sulfuric acid and 30 μL of a 40 g/L ammonium molybdate solution were added, and stirred well. To the resultant solution, 50 μL of a 0.1 M sodium ascorbate solution was added, and stirred again, and then kept warm at 40°C for 20 minutes. Subsequently, absorbance at 880 nm was measured using a spectrophotometer (UV-1240, manufactured by SHIMADZU CORPORATION), and the concentration (μM) of phosphoric acid was calculated using a calibration curve prepared in advance using potassium dihydrogenphosphate. An activity level was calculated using the following formula.

$$\text{Activity Level (U/mL)} = \text{Phosphoric Acid Concentration (μM)} \times \text{Reaction Liquid Amount (L)} / \text{Reaction time (minute)} / \text{Enzyme Amount (mL)} \times \text{Dilution Rate}$$

a Temperature dependency was determined in such a manner that PPase activity at various temperatures, namely, room temperature (23.5°C), 37°C, 43°C, 50°C, and 60°C was measured.

b Temperature stability was determined in such a manner that a PPase sample was kept warm for 2 hours at various temperatures, namely, 4°C, 25°C, 37°C, 43°C, 50°C, and 60°C, and centrifuged on the condition of 15000 r/min and 4°C, and the remaining activity of the resultant supernatant was measured.

c pH dependency was determined in such a manner that a liquid substrate having a casein concentration 3 times as high as an original concentration was diluted by 3 times with each of 0.2 M acetic acid-potassium acetate buffer solutions (pH 3.6, 4.2, 4.7, 5.2, and 5.6), MES-NaOH buffer solutions (pH 5.3, 6.0, and 6.8), Tris-HCl buffer solutions (pH 7.0, 7.5, 8.0, and 9.0), and left to stand at 4°C for 6 hours. Then the remaining activity of each of the obtained samples was measured. Note that a pH after diluting the substrate with each of the buffer solutions was measured, and the measured pH value was regarded as a reaction pH.

d pH stability was determined in such a manner that dilution by 10 times with each of 0.2 M acetic acid-potassium acetate buffer solutions (pH 3.6, 4.2, 4.7, 5.6, and 6.0), MES-NaOH buffer solutions (pH 5.3, 6.0, and 6.8), Tris-HCl buffer solutions (pH 7.5, 8.0, and 9.0) was performed. Each of the resultant samples was left to stand at 4°C for 6 hours, and then the remaining activity of each of the samples was measured.

e Metal ion requirement was determined in such a manner that each of sodium chloride, potassium chloride, lithium chloride, calcium chloride, cobalt chloride, and manganese chloride and an EDTA·2 sodium solution was added so as to achieve 2.5 mM, 5 mM, and 10 mM in a reaction liquid, and PPase activity at this point in time was measured.

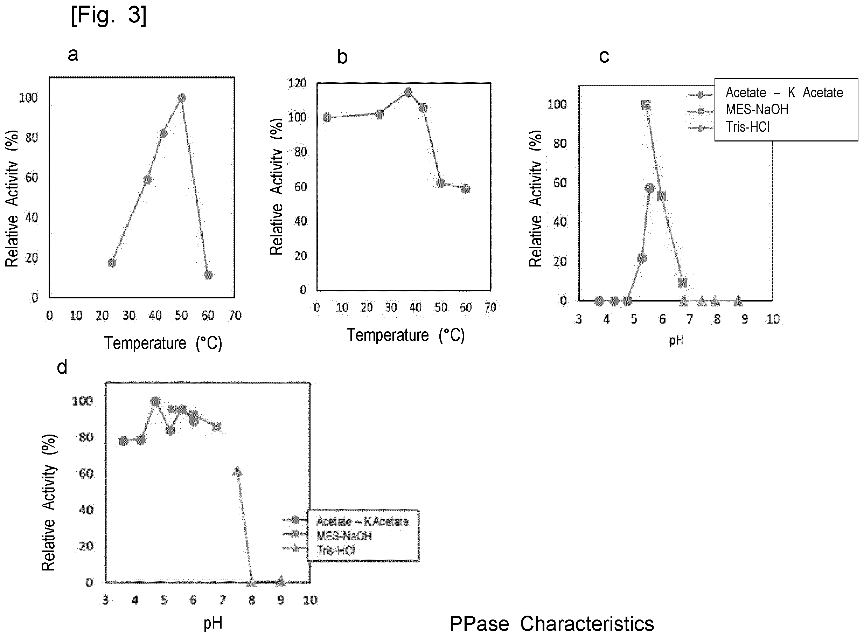

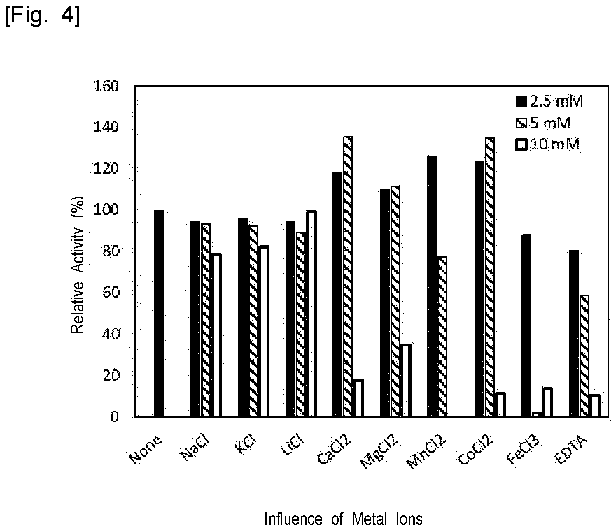

FIG. 3a shows the temperature dependency of PPase, FIG. 3b shows the temperature stability thereof, FIG. 3c shows the pH dependency thereof, FIG. 3d shows the pH stability thereof, and FIG. 4 shows the metal ion requirement thereof. From FIGS. 3a to 3d, it was confirmed that, for the PPase, the optimum temperature was 50°C and the optimum pH was pH 5.42, and the PPase maintained 80% or higher of the remaining activity at 43°C for 2 hours, and at pH 4.7 to 6.8 for 6 hours.

In FIG. 4, it was confirmed that the PPase was activated with 2.5 to 5.0 mM bivalent metal cations, such as $Ca^{2+}$, $Mg^{2+}$, $Mn^{2+}$, and $Co^{2+}$.

Example 1(1) Preparation of Fermented Milk

2% skim milk (manufactured by MORINAGA MILK INDUSTRY CO., LTD.) was added to "Oishii Gyunyu" (manufactured by Meiji Co., Ltd.), and shaken well to be dissolved. The resultant raw material milk was sterilized in boiling water while stirring for 20 minutes. The raw material milk after the sterilization was kept warm at 43°C for approximately 30 minutes, and subsequently, a starter L812 (manufactured by Chr.Hansen) and PPase or a 20 mM acetic acid-potassium acetate buffer solution (pH 5.2) illustrated in Table 1 were added thereto so as to achieve a final concentration of 0 to 10 U/mL-milk in enzyme activity, and the resultant mixture was fermented at 43°C for 4.5 hours, whereby fermented milk was obtained.

Note that the PPase to be used was suitably diluted with a 20 mM acetic acid-potassium acetate buffer solution (pH 5.2). The amount of casein in the fermented milk in Example 1 was 3.3g/100g.

[Table 1]

| Experiment | Raw material milk (mL) | PPase (mL) | Acetic acid buffer solution (mL) | Starter (mL) | PPase final concentration (U/ml-milk) |
|---|---|---|---|---|---|
| 1 | 12 | 0 | 0.364 | 10 | 0 |
| 2 | 12 | 0.364 | 0 | 10 | 1 |
| 3 | 12 | 0.364 | 0 | 10 | 2 |
| 4 | 12 | 0.364 | 0 | 10 | 3 |
| 5 | 12 | 0.364 | 0 | 10 | 5 |
| 6 | 12 | 0.364 | 0 | 10 | 10 |

(2) Measurement of Dephosphorylated Casein

The fermented milk was diluted by 50 times with distilled water and mixed with an equivalent amount of a × 2 sample buffer. The resultant mixture was heated for 5 minutes in boiling water to obtain a measurement sample. 10 μL of the measurement sample was applied to SuperSepTM Phos-tag (12.5%, manufactured by FUJIFILM Wako Pure Chemical Corporation) and subjected to electrophoresis at a constant current of 25 mM. A gel obtained after the electrophoresis was dyed and decolorized, and then an electrophoresis pattern was graphed using image-processing software (Image J). Subsequently, regions varying with the presence or absence of phosphoric acid modification were set, and the area of each of the regions was calculated. Subsequently, the ratio of dephosphorylated casein and the amount (absolute amount) of the dephosphorylated casein with respect to enzyme amounts were calculated.

(3) Measurement of Free Phosphoric Acid Concentration (mM)

The fermented milk prepared in the above-described (1) was shaken violently to make a curd uniform. 1 mL of the fermented milk in which the curd was uniform was dispensed into a 1.5-mL Eppendorf tube, and centrifuged for 10 minutes on the condition of 15000 r/min and 4°C. 200 μL of a suitably diluted solution of the resultant supernatant was added to 800 μL of distilled water, and subsequently 2M sulfuric acid and 30 μL of a 40 g/L ammonium molybdate solution were added, and stirred well. To the resultant solution, 50 μL of a 0.1 M sodium ascorbate solution was added, and stirred again, and then kept warm at 40°C for 20 minutes. Subsequently, absorbance at 880 nm was measured using a spectrophotometer (UV-1240, manufactured by SHIMADZU CORPORATION), and the concentration (mM) of phosphoric acid was calculated using a calibration curve prepared in advance using potassium dihydrogenphosphate.

(4) Results

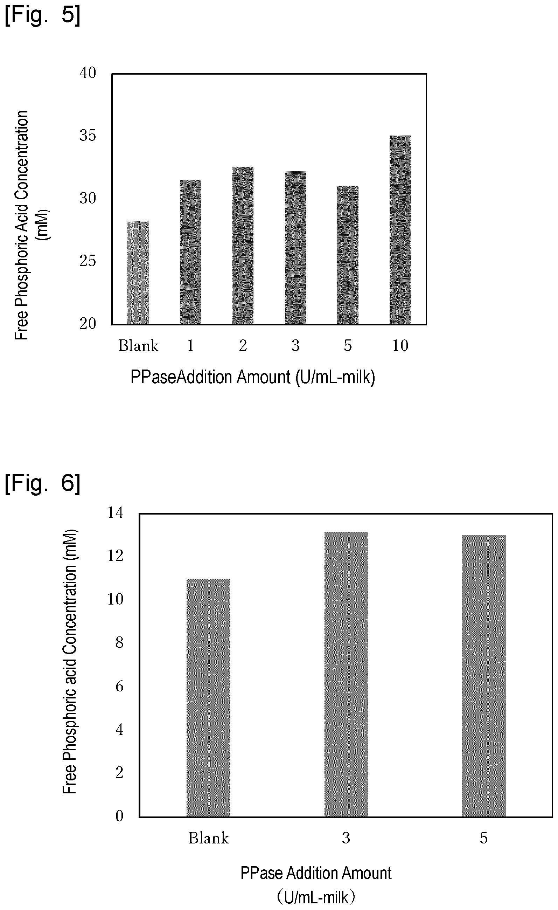

Table 2 shows the measurement results on the dephosphorylated casein. FIG. 5 shows the measurement results on the concentration of the free phosphoric acid.

[Table 2]

| | PPase final concentration (U/mL-milk) | α | | β | | κ | | All types of casein | |
|---|---|---|---|---|---|---|---|---|---|
| | | Ratio (%) | Absolute amount (g/100g) | Ratio (%) | Absolute amount (g/100g) | Ratio (%) | Absolute amount (g/100g) | Ratio (%) | Absolute amount (g/100g) |
| Fermented milk | 0 | 25.1 | 0.4 | 13.6 | 0.1 | 25.8 | 0.1 | 21.5 | 0.7 |
| | 1 | 28.8 | 0.5 | 24.8 | 0.3 | 18.2 | 0.1 | 26.3 | 0.9 |
| | 2 | 28.6 | 0.5 | 27.6 | 0.3 | 21.7 | 0.1 | 27.5 | 0.9 |
| | 3 | 35.8 | 0.6 | 46.0 | 0.5 | 32.0 | 0.1 | 39.8 | 1.3 |
| | 5 | 38.9 | 0.7 | 46.2 | 0.5 | 26.0 | 0.1 | 40.2 | 1.3 |
| | 10 | 40.3 | 0.7 | 45.1 | 0.5 | 29.6 | 0.1 | 40.9 | 1.3 |

Example 2

(1) Preparation of Dephosphorylated Milk

300 U/mL of PPase was added in an amount illustrated in Table 3 to "Oishii Gyunyu" (manufactured by Meiji Co., Ltd.) having a pH of 5.9 adjusted with 1N HCl. The resultant mixture was left to stand at 43°C for 4 hours to obtain dephosphorylated milk. The amount of casein in the dephosphorylated milk of Example 2 was 2.5 g / 100 g.

[Table 3]

| Experiment | Raw material milk (mL) | PPase (μL) | Water (μL) | PPase final concentration (U/ml-milk) |
|---|---|---|---|---|
| 1 | 0.5 | 0 | 50 | 0 |
| 2 | 0.5 | 5 | 45 | 3 |
| 3 | 0.5 | 8.3 | 41.7 | 5 |

(2) Measurement of Dephosphorylated Casein

Dephosphorylated casein was measured using the same method as in Example 1, except that dephosphorylated milk on which PPase acted was used.

(3) Measurement of Free Phosphoric Acid Concentration (mM)

The concentration of free phosphoric acid was measured using the same method as in Example 1, except that dephosphorylated milk on which PPase acted was used.

(4) Results

Table 4 shows the measurement results on the dephosphorylated casein. FIG. 6 shows the measurement results on the concentration of the free phosphoric acid.

[Table 4]

| | PPase final concentration (U/mL-milk) | α | | β | | K | | All types of casein | |
|---|---|---|---|---|---|---|---|---|---|
| | | Ratio (%) | Absolute amount (g/100g) | Ratio (%) | Absolute amount (g/100g) | Ratio (%) | Absolute amount (g/100g) | Ratio (%) | Absolute amount (g/100g) |
| Dephosphorylated milk | 0 | 31.4 | 0.4 | 32.5 | 0.3 | 6.9 | 0.0 | 28.4 | 0.7 |
| | 3 | 50.8 | 0.6 | 54.2 | 0.4 | 15.6 | 0.0 | 48.2 | 1.1 |
| | 5 | 55.1 | 0.7 | 81.5 | 0.6 | 40.6 | 0.1 | 61.7 | 1.4 |

Example 3

(1) Preparation of Fermented Milk

392 g of low-temperature pasteurized milk (manufactured by Takanashi Milk Products Co., Ltd.) and 8 g of skim milk (manufactured by MORINAGA MILK INDUSTRY CO., LTD.) were weighed out in a 500-mL medium bottle, and shaken well to be dissolved. The resultant raw material milk was sterilized in boiling water while stirring for 20 minutes. The raw material milk after the sterilization was kept warm at 43°C for approximately 30 minutes, and subsequently a starter L812 (manufactured by Chr.Hansen, hereinafter the same) was added thereto to achieve 0.01 g/mL, and mixed by gently inverting the bottle approximately 10 times. Subsequently, 10 mL of the raw material milk to which the starter was added was dispensed into each of 15-mL screw-cap tubes to which samples illustrated in Table 5 were respectively added in advance. With the tube covered with the cap, the mixture was mixed by gently inverting the tube 2 to 3 times, and subsequently fermented at 43°C for 5 hours. After the fermentation, cooling was performed at 4°C overnight to obtain fermented milk. The pH of the fermented milk was measured using a pH meter (F72-S, manufactured by HORIBA, Ltd).

[Table 5]

| Samples added to fermented milk | | |
| --- | --- | --- |
| Sample | Addition amount (mL) | Sample final concentration |
| Blank (20 mL acetic acid-potassium acetate buffer solution (pH 5.2)) | 0.48 | - |
| Purified PPase (11 U/mL) | 0.48 | 0.53 U/mL-milk |
| Phosphoric acid (1.47 M) | 0.0068+Buf.0.4732 | 1 mM |
| | 0.034+Buf.0.446 | 5 mM |
| | 0.068+Buf.0.412 | 10 mM |

(2) Measurement of Water Weeping Percentage

The fermented milk sample prepared in the above-described (1) was shaken violently to make a curd uniform. Approximately 5 mL of the fermented milk was dispensed into a 15-mL graduated screw-cap tube, and centrifuged for 10 minutes on the condition of 3000 r/min (H-19F MR, manufactured by KOKUSAN Co. Ltd.) and 8°C. Subsequently, the whole volume of the sample and the volume of solids were read out, and water weeping percentage was calculated by the following formula.

```
Water Weeping Percentage (%) = 100 - ((solids volume / sample
volume) × 100)
```

(3) Measurement of Free Phosphoric Acid Concentration (mM)

1 mL of the fermented milk sample in which the curd was uniform was dispensed into a 1.5-mL Eppendorf tube, and centrifuged for 10 minutes on the condition of 15000 r/min (MRX-150, high speed refrigerated centrifuge, manufactured by TOMY SEIKO CO., LTD.) and 4°C. 200 μL of a suitably diluted solution of the resultant supernatant was added to 800 μL of distilled water, and subsequently 2M sulfuric acid and 30 μL of a 40 g/L ammonium molybdate solution were added, and stirred well. To the resultant solution, 50 μL of a 0.1 M sodium ascorbate solution was added, and stirred again, and then kept warm at 40°C for 20 minutes. Subsequently, absorbance at 880 nm was measured using a spectrophotometer (UV-1240, manufactured by SHIMADZU CORPORATION), and the concentration (mM) of phosphoric acid was calculated using a calibration curve produced in advance using potassium dihydrogenphosphate.

(4) Results

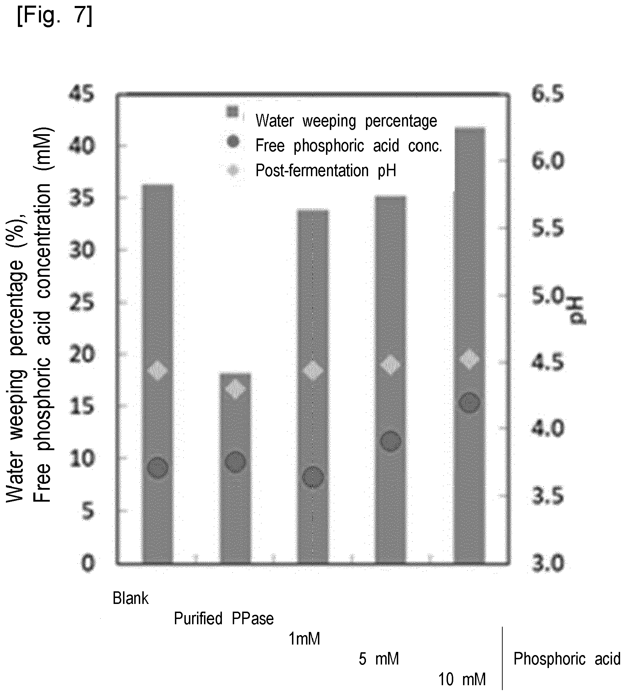

FIG. 7 shows the results. FIG. 7 revealed that the fermented milk to which PPase was added exhibited lower water weeping percentage than in PPase-additive-free milk (Blank). In contrast, in the fermented milk containing phosphoric acid as a reaction product of PPase, but not containing dephosphorylated casein, water weeping was not suppressed.

FIG. 7 revealed that the addition of PPase during fermentation was more effective for suppressing water weeping than in PPase-additive-free milk (Blank). In contrast, even when phosphoric acid as a reaction product of PPase was added, water weeping was not suppressed.

Example 4

(1) Preparation of Fermented Milk

392 g of low-temperature pasteurized milk (manufactured by Takanashi Milk Products Co., Ltd.) and 8 g of skim milk (manufactured by MORINAGA MILK INDUSTRY CO., LTD.) were weighed out in a 500-mL medium bottle, and shaken well to be dissolved. The resultant raw material milk was heat-sterilized in boiling water for 20 minutes, and then kept warm at 43°C for approximately 30 minutes. To the resultant, 40 mg of a starter L812 was added, and mixed by gently inverting the bottle 10 times. Subsequently, 60 g of the raw material milk was weighed out in each of jam bottles into which 1.46 mL of suitably diluted purified PPase (1.0 U/mL-milk or 3.0 U/mL-milk) or a 20 mM acetic acid-potassium acetate buffer solution (pH 5.2) was dispensed in advance. Mixing was performed by inverting the bottle 5 times, and subsequently the resultant mixture was fermented at 43°C for 4.5 hours. After the fermentation, cooling was performed at 4°C overnight to obtain fermented milk. The pH of the fermented milk was measured using a pH meter (ORION 3 STAR, manufactured by Thermo Fisher Scientific K.K).

(2) Measurement of Water Weeping Percentage

Water weeping percentage was measured by the same method as in Example 3.

(3) Measurement of Free Phosphoric Acid Concentration (mM)

The concentration of free phosphoric acid was measured by the same method as in Example 3.

(4) Measurement of EPS

0.4 g of a fermented milk sample was weighed out, and 200 $\mu$L of a 100% (w/v) TCA solution was added thereto, and mixed by inverting. The resultant mixture was centrifuged for 10 minutes on the condition of 15000 r/min (MRX-150, high speed refrigerated centrifuge, manufactured by TOMY SEIKO CO., LTD., hereinafter the same) and 4°C. The whole amount of the resultant supernatant was collected into a new Eppendorf tube, and 500 $\mu$L of acetone was added thereto. The resultant mixture was left to stand at 4°C overnight. Subsequently, centrifugation was conducted for 10 minutes on the condition of 15000 r/min and 4°C, and the resultant supernatant was discarded. Subsequently, a precipitated pellet was dissolved in 400 $\mu$L of MilliQ water, and then 400 $\mu$L of acetone was added thereto and mixed by inverting the tube. The resultant mixture was left to stand at 4°C overnight. Subsequently, centrifugation was conducted for 10 minutes on the condition of 15000 r/min and 4°C, and the resultant supernatant was discarded. Then, a precipitate was dissolved in 300 $\mu$L of MilliQ. The resultant solution was centrifuged for 10 minutes on the condition of 15000 r/min and 4°C, whereby a supernatant was collected. Using 100 $\mu$L of the solution, the amount of monosaccharide and disaccharide was measured by HPLC (manufactured by Waters Corporation). Furthermore, using the remaining 200 $\mu$L of the solution, the total amount of saccharides was measured by a phenol-sulfuric acid method. By subtracting the amount of the monosaccharide and the disaccharide from the total amount of saccharide, the amount of extracellular polysaccharides, that is, trisaccharides or higher was calculated.

(5) Measurement of Viscosity

A fermented milk sample was violently shaken to be uniform, and 13.5 g of the sample was dispensed into a disposable sample chamber (made of aluminum) for a viscometer (DV-I Prime, BROOK FIERD). Using SC4-29 as a spindle, viscosity was measured at 10 r/min every 30 seconds and recorded. Note that all the operations were performed under an environment of 10°C.

(6) Analysis on Breaking Strength

A device used for the analysis was a creep meter RE2-33005C (manufactured by YAMADEN co.,ltd.) and the plunger No.3 was used.

Measurements were performed on the condition of a load cell of 0.01, an amplifier magnification of 0.1, a storage pitch of 0.07 sec, a measured strain rate of 60%, a measurement speed of 1 mm/sec, and automatic measurement applied for sample thickness.

(7) Results

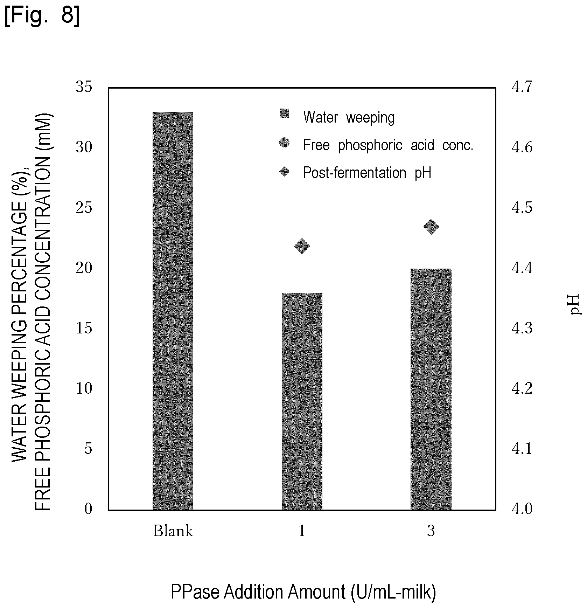

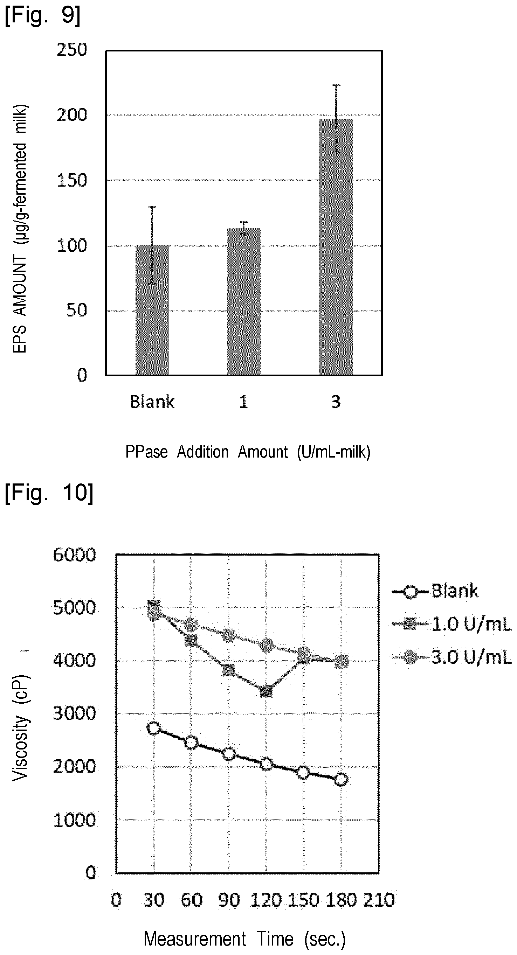

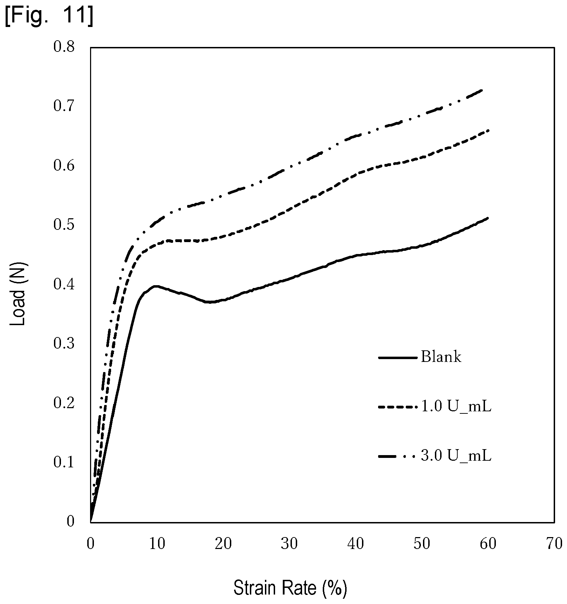

FIG. 8 shows the measurement results on water weeping percentage, free phosphoric acid concentration, and post-fermentation pH. FIG. 9 shows EPS concentration. FIG. 10 shows the measurement results on viscosity. FIG. 11 shows the analysis results on breaking strength.

The fermented milk containing dephosphorylated casein exhibited lower water weeping percentage than PPase-additive-free (Blank) (see FIG. 8), and the fermented milk had a larger amount of extracellular polysaccharides (EPS) than the Blank (see FIG. 9). The viscosity of the fermented milk was higher by 1000 cP or more than that of the PPase-additive-free milk (see FIG. 10). Furthermore, the breaking stress of the fermented milk was higher than that of the PPase-additive-free milk, so that a decline of the stress (= fragility) after breaking was reduced (see FIG. 11).

Example 5

(1) Preparation of Fermented Milk

147 g of low-temperature pasteurized milk (manufactured by Takanashi Milk Products Co., Ltd.) and 3 g of skim milk (manufactured by MORINAGA MILK INDUSTRY CO., LTD.) were weighed out in each of five 250-mL medium bottles, and shaken well to be dissolved. Subsequently, the resultant was heat-sterilized in boiling water for 20 minutes, and then kept warm at 43°C for approximately 30 minutes. To the resultant, various types of starters illustrated in Table 6 (all manufactured by Chr. Hansen) were added, and mixed by gently inverting the bottle 10 times. Subsequently, 60 g of the resultant raw material milk was weighed out in each of jam bottles into which 0.488 mL of 123 U/mL purified PPase (Final 1.0 U/mL-milk) or a 20 mM acetic acid-potassium acetate buffer solution (pH 5.2) was dispensed in advance. The resultant mixture was mixed by inverting the bottle 5 times, and then fermented at 43°C for 4.5 hours. After the fermentation, cooling was performed at 4°C overnight to obtain fermented milk.

[Table 6]

| Starter's Name and Addition Amount | |
| --- | --- |
| Starter | Addition Amount |
| L811 | 15 mg |
| L812 | 15 mg |
| Harmony | 30 mg |

(2) Measurement of Dephosphorylated Casein, (3) Measurement of Water Weeping Percentage, (4) Measurement of Free Phosphoric Acid Concentration (mM), (5) Measurement of EPS, (6) Measurement of Viscosity, (7) Analysis on Breaking Strength

These measurements were performed using the same methods as in Example 4.

(8) Results

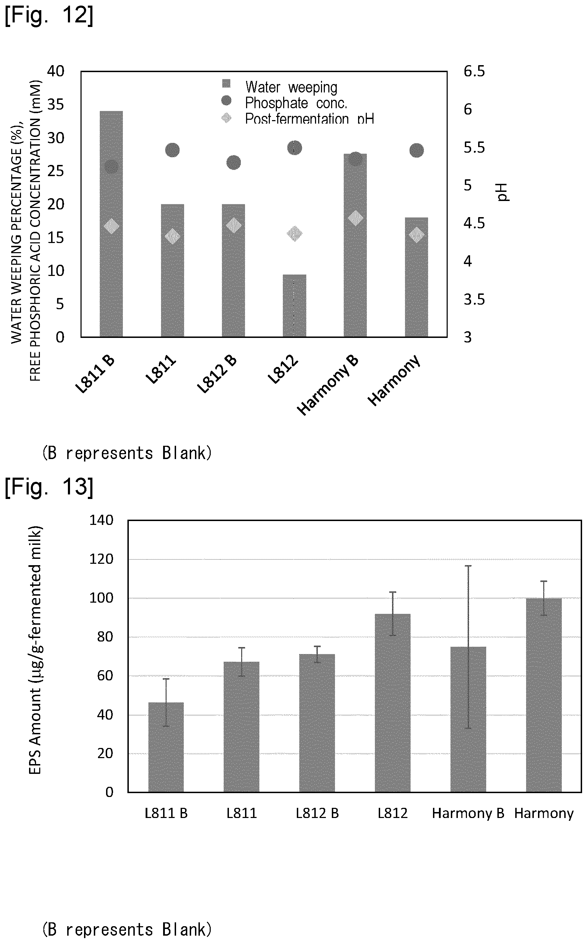

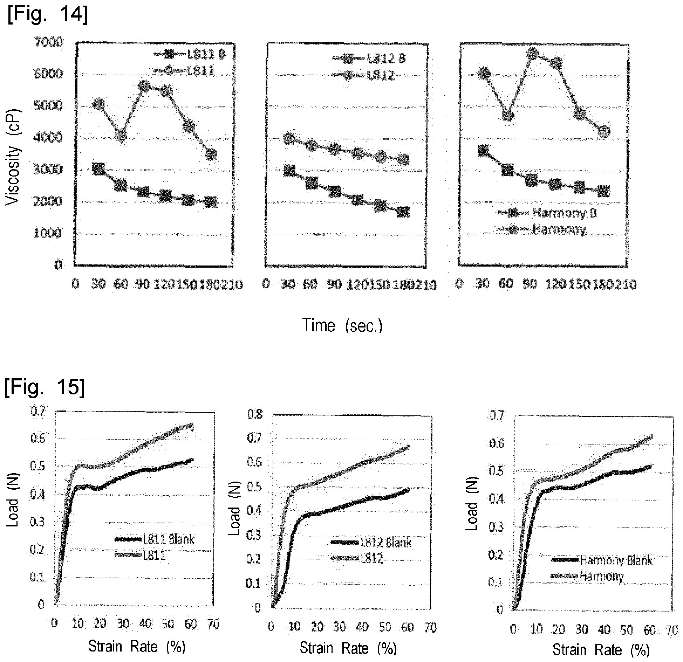

FIG. 12 shows the measurement results on water weeping percentage, free phosphoric acid concentration, and post-fermentation pH. FIG. 13 shows the concentration of EPS. FIG. 14 shows the measurement results on viscosity. FIG. 15 shows the analysis results on breaking strength.

The fermented milk containing dephosphorylated casein exhibited lower water weeping percentage than PPase-additive-free milk (Blank) (see FIG. 12), and the fermented milk had a larger amount of extracellular polysaccharides (EPS) than the PPase-additive-free milk (see FIG. 13). The viscosity of the fermented milk was higher by 1000 cP to 2000 cP than that of the PPase-additive-free milk (see FIG. 14). Furthermore, the breaking stress of the fermented milk was higher than that of the PPase-additive-free milk (see FIG. 15).

It was observed that the addition of PPase caused water weeping to be more suppressed, the amount of free phosphoric acid to be larger, and pH to be lower in all the starters, compared with PPase-additive-free milk (Blank) (see FIG. 12). In all the starters, the amount of extracellular polysaccharides (EPS) was larger than in the PPase-additive-free milk (see FIG. 13). Measurement results on the viscosity of the fermented milk revealed that the viscosity increased by 1000 cP to 2000 cP (see FIG. 14). As a result of analysis on the breaking strength of the fermented milk, it was confirmed that breaking stress increased in all the starters (see FIG. 15).

Example 6

(1) Preparation of Fermented Milk

392 g of low-temperature pasteurized milk (manufactured by Takanashi Milk Products Co., Ltd.) and 8 g of skim milk (manufactured by MORINAGA MILK INDUSTRY CO., LTD.) were weighed out in a 500-mL medium bottle, and shaken well to be dissolved. Subsequently, the resultant was heat-sterilized in boiling water for 20 minutes, and then kept warm at 43°C for approximately 30 minutes. To the resultant, 40 mg of a starter L812 was added, and mixed by gently inverting the bottle 10 times. Subsequently, 60 g of the resultant raw material milk was weighed out in each of jam bottles into which 1.46 mL of suitably diluted purified PPase or a 20 mM acetic acid-potassium acetate buffer solution (pH 5.2) was dispensed in advance. Mixing was performed by inverting the bottle 5 times, and subsequently the resultant mixture was fermented at 43°C for 4.5 hours. After the fermentation, cooling was performed at 4°C overnight to obtain fermented milk. The pH of the fermented milk was measured using a pH meter (ORION 3 STAR, manufactured by Thermo Fisher Scientific K.K).

(2) Measurement of Water Weeping Percentage, (3) Measurement of Free Phosphoric Acid Concentration (mM), (4) Measurement of EPS

These measurements were performed using the same methods as in Example 4.

(5) Measurement of Viscosity

A fermented milk sample was violently shaken to be uniform, and 13.5 g of the sample was dispensed into a disposable sample chamber (made of aluminum) for a viscometer (DV-I Prime, BROOK FIERD). Using SC4-29 as a spindle, viscosity was measured at 10 r/min every 30 seconds and recorded. Note that all the operations were performed under an environment of 10°C.

(6) Analysis on Breaking Strength

A device used for the analysis was a creep meter RE2-33005C (manufactured by YAMADEN co.,ltd.) and the plunger No.3 was used. Measurements were performed on the condition of a load cell of 0.01, an amplifier magnification of 0.1, a storage pitch of 0.07 sec, a measurement strain rate of 60%, a measurement speed of 1 mm/sec, and automatic measurement applied for sample thickness.

(7) Results

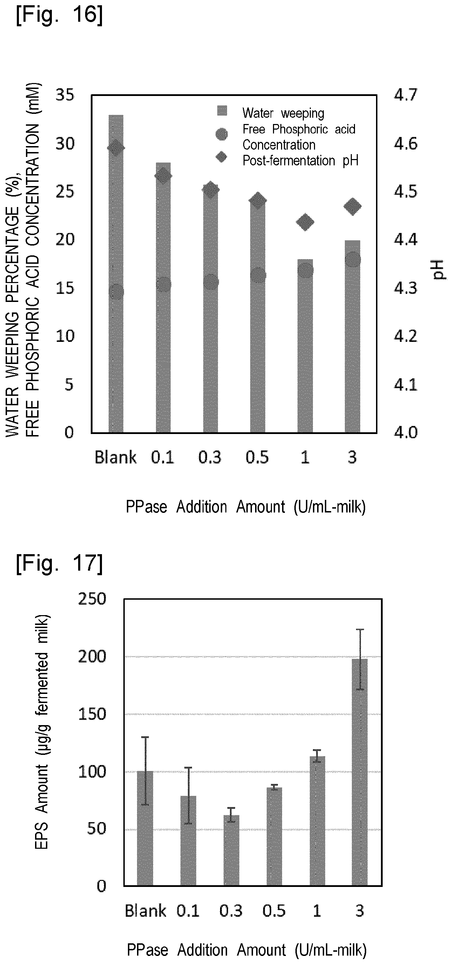

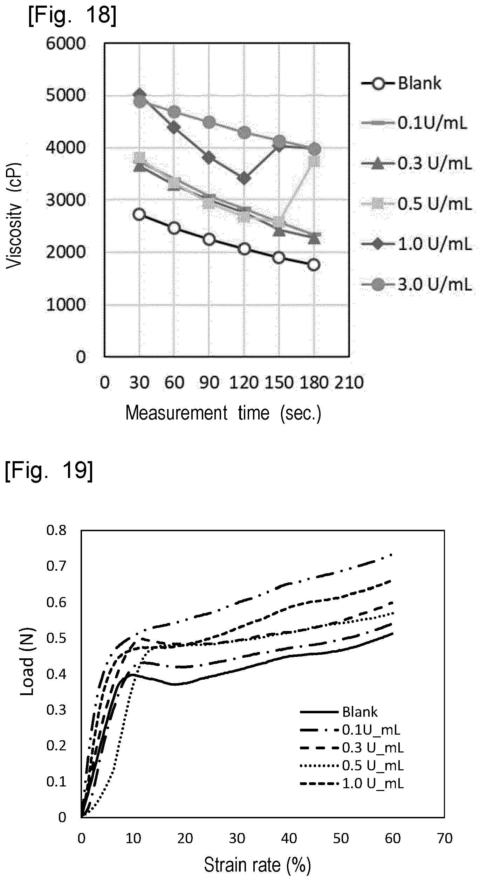

FIG. 16 shows the measurement results on water weeping percentage, free phosphoric acid concentration, and post-fermentation pH. FIG. 17 shows EPS concentration. FIG. 18 shows the measurement results on viscosity. FIG. 19 shows the analysis results on breaking strength. When PPase was added to the fermented milk in a range of 0.1 to 3.0 U/mL-milk, the fermented milk exhibited lower water weeping percentage, a larger amount of free phosphoric acid, and a lower pH than the PPase-additive-free milk (Blank) (see FIG. 16). With the addition of 1.0 and 3.0 U/mL-milk PPase, the fermented milk had a larger amount of extracellular polysaccharides (EPS) than the PPase-additive-free milk (see FIG. 17). Measurement results on the viscosity of the fermented milk to which PPase was added revealed that, with the addition of 0.1 to 0.5 U/mL-milk PPase, the viscosity of the fermented milk was higher by approximately 1000 cP than the viscosity of the PPase-additive-free milk, meanwhile, with the addition of 1.0 and 3.0 U/mL-milk PPase, the viscosity of the fermented milk was still higher than the viscosity of the PPase-additive-free milk (see FIG. 18). Analysis results on the breaking strength of the fermented milk to which PPase was added revealed that, although concentration dependency was not observed, the fermented milk had greater breaking stress than the PPase-additive-free milk. In the fermented milk to which 0.5 U/mL-milk PPase was added, a decline of the stress (= fragility) after breaking was reduced (see FIG. 19).

Example 7 (Enzyme Treatment to Raw Material Milk)

(1) Preparation of Fermented Milk

392 g of ultra-high temperature sterilized milk ("Oishii Gyunyu", manufactured by Meiji Co., Ltd.) or low-temperature pasteurized milk (manufactured by Takanashi Milk Products Co., Ltd.) and 8 g of skim milk (manufactured by MORINAGA MILK INDUSTRY CO., LTD.) were weighed out in a 500-mL medium bottle, and shaken well to be dissolved. To the resultant, 2 mL of purified PPase (700 U/mL, Final 23.3 U/mL-milk) or a 20 mM acetic acid-potassium acetate buffer solution (pH 5.2) was added, and mixed by inverting the bottle 5 times. Subsequently, 60 g of the resultant raw material milk was weighed out in each of jam bottles, and mixed by inverting the bottle 5 times. Subsequently the resultant mixture was left to stand at 37°C for 3 hours to obtain enzyme-treated milk. Each of the jam bottles was boiled in boiling water for 20 minutes to deactivate the PPase. Subsequently, the enzyme-treated milk was cooled to room temperature, and then 20 mg of a starter L812 was added into each of the jam bottles. The resultant was mixed by gently inverting the bottle 10 times, and fermented at 43°C for 4.5 hours. After the fermentation, cooling was performed at 4°C overnight to obtain fermented milk. The pH of the fermented milk was measured using a pH meter (ORION 3 STAR, manufactured by Thermo Fisher Scientific K.K).

(2) Measurement of Water Weeping Percentage, (3) Measurement of Free Phosphoric Acid Concentration (mM)

These measurements were performed using the same methods as in Example 4.

(4) Results

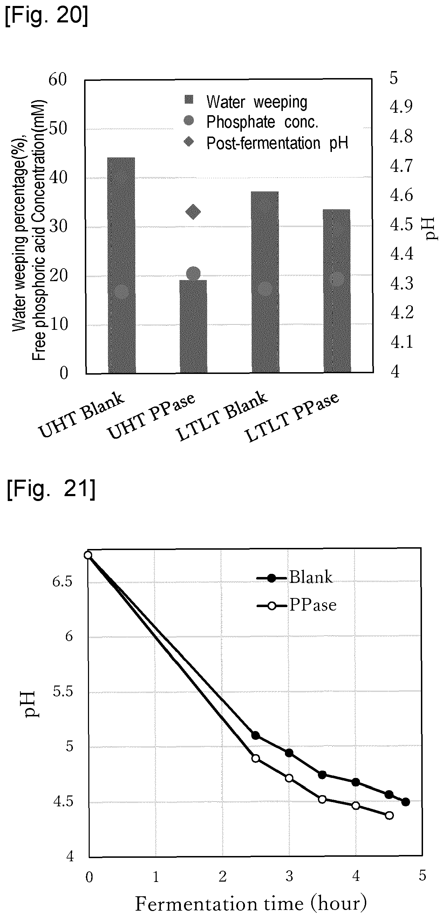

FIG. 20 shows the measurement results on water weeping percentage, free phosphoric acid concentration, and post-fermentation pH. The fermentation of the PPase-treated raw material milk caused the fermented milk to exhibit lower water weeping percentage, a larger amount of free phosphoric acid, and a lower pH than the PPase-additive-free milk (Blank) (see FIG. 20). Such results indicated that casein contained in the milk is beneficially dephosphorylated and, during fermentation or in the fermented milk, PPase is not necessarily present in an active state.

Example 8

(1) Preparation of Fermented Milk

392 g of ultra-high temperature sterilized milk ("Oishii Gyunyu", manufactured by Meiji Co., Ltd.) and 8 g of skim milk (manufactured by MORINAGA MILK INDUSTRY CO., LTD.) were weighed out in a 500-mL medium bottle, and shaken well to be dissolved. The resultant raw material milk was sterilized in boiling water while stirring for 20 minutes. The raw material milk after the sterilization was kept warm at 43°C for approximately 30 minutes, and subsequently 0.571 mL of purified PPase (350 U/mL, Final 1 U/mL-milk) or a 20 mM acetic acid-potassium acetate buffer solution (pH 4.5) and 20 mg of a starter L812 were added thereto, and mixed by gently inverting the bottle 10 times. 10 g of the resultant mixture was dispensed into each of eight 15-mL screw-cap tubes. With the tube covered with the cap, mixing was performed by gently inverting the tube 2 to 3 times, and subsequently the resultant mixture was fermented at 43°C for 5 hours. After the fermentation, cooling was performed at 4°C overnight to obtain fermented milk. The pH of the fermented milk was measured before fermentation, and 2.5 hours, 3 hours, 3.5 hours, 4 hours, and 4.5 hours after the start of the fermentation by using a pH meter (ORION 3 STAR, manufactured by Thermo Fisher Scientific K.K).

(2) Results

FIG. 21 shows the results. It was confirmed that, in the fermented milk to which PPase was added, the pH declined earlier than in the PPase-additive-free milk (Blank) to which the acetic acid-potassium acetate buffer solution was added. It was supposed that phosphoric acid increased due to the addition of PPase. It was also confirmed that the fermented milk to which PPase was added coagulated earlier.

Example 9 (Fermentation time was adjusted so as to achieve a pH of approximately 4.5 after fermentation)

(1) Preparation of Fermented Milk

Fermented milk was prepared in the same manner as in Example 8, except that 60 g of the mixture was dispensed to each of jam bottles in place of each of the 15-mL screw-cap tubes, and, in order to uniformize post-fermentation pHs, the fermentation time of a system to which the buffer solution was added was 4.75 hours, and the fermentation time of a system to which PPase was added was 3.5 hours. The pH of the fermented milk was measured using a pH meter (ORION 3 STAR, manufactured by Thermo Fisher Scientific K.K) .

(2) Measurement of Water Weeping Percentage, (3) Measurement of Free Phosphoric Acid Concentration (mM)

These measurements were performed using the same methods as in Example 4.

(4) Results

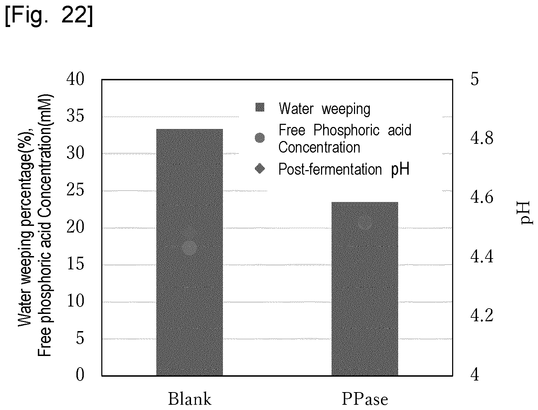

FIG. 22 shows the results. Although the fermented milk and the PPase-additive-free milk (Blank) had the same pH approximately 4.5 after fermentation, the addition of PPase during fermentation caused the fermented milk to exhibit lower water weeping percentage and a larger amount of free phosphoric acid than the PPase-additive-free milk (Blank). This indicated that a decrease in water weeping percentage and an increase in the amount of free phosphoric acid was affected not by post-fermentation pH, but by the action of PPase.

Reference Example (Free Phosphoric Acid Concentration and Calcium Ion Concentration in Enzyme-treated Milk)

(1) Preparation of Enzyme-treated Milk

To 0.45 mL of ultra-high temperature sterilized milk ("Oishii Gyunyu", manufactured by Meiji Co., Ltd., pH 6.76) or milk obtained by adding 1 M hydrochloric acid to the ultra-high temperature sterilized milk to adjust the pH to 5.48, 0.05 mL of purified PPase (700 U/mL) was added. The resultant mixture allowed to react at 37°C for a predetermined time, and subsequently 0.5 mL of 18% trichloroacetic acid was added thereto and mixed to stop the reaction, whereby enzyme-treated milk was obtained.

(2) Measurement of Free Phosphoric Acid Concentration

The concentrations (mM) of free phosphoric acid before and after the enzyme treatment were measured using the same method as in Example 3.

(3) Measurement of Calcium Ion Concentration

Without being diluted, milk before and after the enzyme treatment was placed on a calcium ion sensor (LAQUA twin, manufactured by HORIBA, Ltd.) and measured.

(4) Results

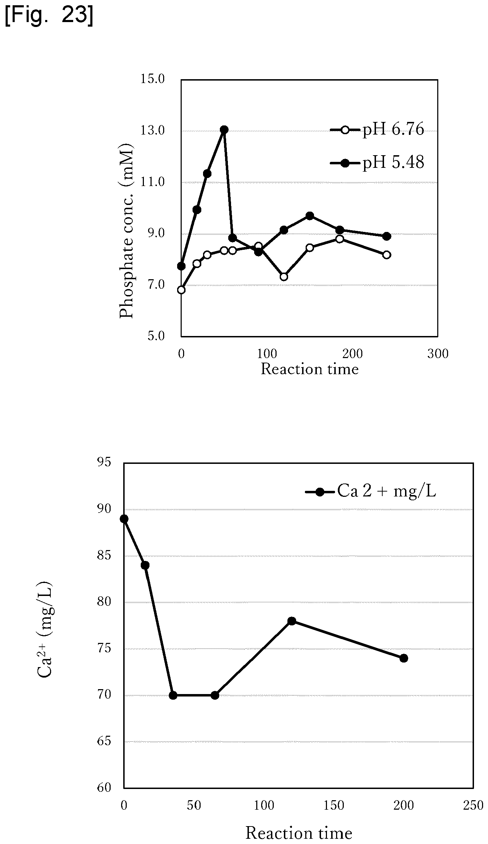

FIG. 23 shows the results. The milk having a pH adjusted to 5.48 exhibited higher PPase reactivity than the milk having a pH of 6.76. In the milk having a pH adjusted to 5.48, the concentration of free phosphoric acid increased temporarily, and then converged to a constant value. In the milk having a pH of 6.76, the concentration of free phosphoric acid increased over time, and then converged to a constant value. In either of the milk to which PPase was added, the concentration of free phosphoric acid converged to a constant value. The concentration of calcium ions changed with the enzyme reaction, which indicated that calcium phosphate was produced in the milk to be in an equilibrium state at a constant concentration.

**Claims**

1. Fermented milk, containing 0.8 g / 100 g or more of dephosphorylated casein.

2. The fermented milk according to claim 1, wherein a content of the dephosphorylated casein is 0.8 g / 100 g to 1.5 g / 100 g.

3. The fermented milk according to claim 1 or 2, wherein a content of free phosphoric acid is 15 mM or more.

4. The fermented milk according to any one of claims 1 to 3, being yogurt.

5. Dephosphorylated milk, containing dephosphorylated casein.

6. The dephosphorylated milk according to claim 5, wherein a content of the dephosphorylated casein is 0.8 g/100 g or more.

7. A method for manufacturing fermented milk, the method including any one of the following steps of:

   (a) fermenting raw material milk in a presence of protein phosphatase; and
   (b) fermenting raw material milk enzyme-treated with protein phosphatase.

8. The method for manufacturing the fermented milk according to claim 7, wherein the protein phosphatase is derived from a microorganism belonging to a genus Trichoderma.

9. The method for manufacturing the fermented milk according to claim 8, wherein the microorganism belonging to the genus Trichoderma is Trichoderma virens.

10. The method for manufacturing the fermented milk according to any one of claims 7 to 9, wherein the protein phosphatase is any one of the following (i) to (iii):

    (i) a protein comprising an amino acid sequence of SEQ ID NO: 2;
    (ii) a protein comprising an amino acid sequence of SEQ ID NO: 2 in which one or several amino acids are deleted, substituted, or added, the protein having protein phosphatase activity; and
    (iii) a protein comprising an amino acid sequence having at least 80% sequence identity with an amino acid sequence of SEQ ID NO: 2, the protein having protein phosphatase activity.

11. The method for manufacturing the fermented milk according to any one of claims 7 to 10, wherein the fermented milk is yogurt.

12. An enzyme agent for fermented milk, the enzyme agent containing protein phosphatase.

13. The enzyme agent for the fermented milk according to claim 12, wherein the protein phosphatase is protein phosphatase derived from a microorganism belonging to a genus Trichoderma.

14. The enzyme agent for the fermented milk according to claim 13, wherein the microorganism belonging to the genus Trichoderma is Trichoderma virens.

15. The enzyme agent for the fermented milk according to any one of claims 12 to 14, wherein the protein phosphatase is any one of the following (i) to (iii):

    (i) a protein comprising an amino acid sequence of SEQ ID NO: 2;
    (ii) a protein comprising an amino acid sequence of SEQ ID NO: 2 in which one or several amino acids are deleted, substituted, or added, the protein having protein phosphatase activity; and
    (iii) a protein comprising an amino acid sequence having at least 80% sequence identity with an amino acid sequence of SEQ ID NO: 2, the protein having protein phosphatase activity.

16. A protein having protein phosphatase activity, the protein being any one of the following (i) to (iii):

    (i) a protein comprising an amino acid sequence of SEQ ID NO: 2;
    (ii) a protein comprising an amino acid sequence of SEQ ID NO: 2 in which one or several amino acids are deleted, substituted, or added, the protein having protein phosphatase activity; and
    (iii) a protein comprising an amino acid sequence having at least 80% sequence identity with an amino acid sequence of SEQ ID NO: 2, the protein having protein phosphatase activity.

[Fig. 1]

ELECTROPHORESIS DIRECTION

[REFERENCE SIGNS LIST]
1  α CASEIN

2  β CASEIN

3  κ CASEIN

11  DEPHOSPHORYLATION REGION OF α CASEIN

21  DEPHOSPHORYLATION REGION OF β CASEIN

31  DEPHOSPHORYLATION REGION OF κ CASEIN

[Fig. 2]

[REFERENCE SIGNS LIST]

100 BAND END (ON ELECTROPHORESIS START SIDE)

110 BASE OF BAND END

200 BAND END (ON ELECTROPHORESIS END SIDE)

210 BASE OF BAND END

A INTEGRATION START LINE

B INTEGRATION END LINE

ELECTROPHORESIS DIRECTION

[Fig. 3]

[Fig. 4]

Influence of Metal Ions

[Fig. 5]

[Fig. 6]

[Fig. 7]

[Fig. 8]

[Fig. 9]

[Fig. 10]

[Fig. 11]

[Fig. 12]

(B represents Blank)

[Fig. 13]

(B represents Blank)

[Fig. 14]

[Fig. 15]

[Fig. 16]

[Fig. 17]

[Fig. 18]

[Fig. 19]

[Fig. 20]

[Fig. 21]

[Fig. 22]

[Fig. 23]

## INTERNATIONAL SEARCH REPORT

| | International application No. |
|---|---|
| | PCT/JP2021/015188 |

**A. CLASSIFICATION OF SUBJECT MATTER**
Int.Cl. A23C9/127(2006.01)i, A23C9/13(2006.01)i, C12N15/55(2006.01)i, C12N9/16(2006.01)i
FI: A23C9/127, C12N15/55ZNA, C12N9/16BZNA, A23C9/13
According to International Patent Classification (IPC) or to both national classification and IPC

**B. FIELDS SEARCHED**

Minimum documentation searched (classification system followed by classification symbols)
Int.Cl. A23C9/127, A23C9/13, C12N15/55, C12N9/16

Documentation searched other than minimum documentation to the extent that such documents are included in the fields searched
Published examined utility model applications of Japan 1922–1996
Published unexamined utility model applications of Japan 1971–2021
Registered utility model specifications of Japan 1996–2021
Published registered utility model applications of Japan 1994–2021

Electronic data base consulted during the international search (name of data base and, where practicable, search terms used)
JSTPlus/JMEDPlus/JST7580(JDreamIII) GenBank/EMBL/DDBJ/GeneSeq,
UniProt/GeneSeq, SwissProt/GeneSeq

**C. DOCUMENTS CONSIDERED TO BE RELEVANT**

| Category* | Citation of document, with indication, where appropriate, of the relevant passages | Relevant to claim No. |
|---|---|---|
| X | JP 48-005913 B2 (MORINAGA MILK INDUSTRY CO., LTD.) 21 February 1973 (1973-02-21), claims, column 2, lines 3-6 | 1-7, 11-12 |
| X<br>Y | UniProtKB, [online], Accession No. G9NAA9, Last modified: 22 February 2012, [retrieved on 08 June 2021], <URL:http://www.uniprot.org/uniprot/G9NAA9>, p. 2 | 16<br>8-11, 13-15 |
| X<br>Y | UniProtKB, [online], Accession No. A0A1T3CP59, Last modified: 10 May 2017, [retrieved on 08 June 2021], <URL:http://www.uniprot.org/uniprot/A0A1T3CP59>, p. 2 | 16<br>8-11, 13-15 |
| X | US 6355297 B1 (N.V. NUTRICIA) 12 March 2002 (2002-03-12), examples 1, 2 | 5-6 |
| Y | CN 105695542 A (JIANGNAN UNIVERSITY) 22 June 2016 (2016-06-22), claims | 1-15 |

☒ Further documents are listed in the continuation of Box C.      ☒ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| 08 June 2021 | 22 June 2021 |

| Name and mailing address of the ISA/<br>Japan Patent Office<br>3-4-3, Kasumigaseki, Chiyoda-ku,<br>Tokyo 100-8915, Japan | Authorized officer<br><br>Telephone No. |
|---|---|

Form PCT/ISA/210 (second sheet) (January 2015)

**INTERNATIONAL SEARCH REPORT**

International application No.

PCT/JP2021/015188

C (Continuation). DOCUMENTS CONSIDERED TO BE RELEVANT

| Category* | Citation of document, with indication, where appropriate, of the relevant passages | Relevant to claim No. |
|---|---|---|
| Y | JP 2017-184682 A (MORINAGA MILK INDUSTRY CO., LTD.) 12 October 2017 (2017-10-12), claims, paragraphs [0004], [0018], [0022], [0023] | 1-15 |
| Y | WO 2017/006926 A1 (GODO SHUSEI KK) 12 January 2017 (2017-01-12), claims, examples | 8-11, 13-15 |

Form PCT/ISA/210 (second sheet) (January 2015)

INTERNATIONAL SEARCH REPORT

Information on patent family members

International application No.

PCT/JP2021/015188

```
JP 48-005913 B2    21 February 1973    (Family: none)

US 6355297 B1      12 March 2002        WO 2000/008946 A1
                                        EP 1104999 A1

CN 105695542 A     22 June 2016         (Family: none)

JP 2017-184682 A   12 October 2017      (Family: none)

WO 2017/006926 A1  12 January 2017      US 2018/0199583 A1
                                        claims, examples
                                        EP 3320781 A1
```

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2015041194 A

**Non-patent literature cited in the description**

- Ministerial Ordinance on Milk and Milk products Concerning Compositional Standards, etc. *Ministry of Health and Welfare Ordinance,* 1951, (52